# EUROPEAN PATENT SPECIFICATION

(11) **EP 3 116 583 B1**
(45) Date of publication and mention of the grant of the patent: **06.03.2024**
(21) Application number: 15760826.6
(22) Date of filing: 12.03.2015
(51) Int. Cl.: A61F 2/04, A61F 2/86, A61F 2/89, A61F 2/848

(54) **INDWELLING BODY LUMEN EXPANDER**
INNEWOHNENDER KÖRPERLUMENEXPANDER
DISPOSITIF D'EXPANSION DE LUMIÈRE À DEMEURE

(30) Priority: 14.03.2014 US 201461953212 P; 11.08.2014 US 201462035826 P
(43) Date of publication of application: 18.01.2017
(73) Proprietor: The Board of Trustees of the Leland Stanford Junior University, Stanford, CA 94305-2038 (US)
(72) Inventor: CHAO, Tiffany E., Boston, Massachusetts 02114 (US); DAMIANO, Nicholas R., San Francisco, California 94131 (US); MEHTA, Shreya, San Francisco, California 94110 (US); WOOCK, John P., Irvine, CA 92618 (US)
(74) Representative: J A Kemp LLP
(86) International application number: PCT/US2015/020235
(87) International publication number: WO 2015/138763

(56) References cited:
- EP-A1- 0 631 762
- WO-A1-2007/048437
- WO-A2-2010/124126
- US-A- 5 269 802
- US-A- 5 540 713
- US-A1- 2004 078 088
- US-A1- 2012 083 820
- US-B2- 7 998 187
- US-B2- 8 282 678

## Description

### FIELD OF THE INVENTION

The invention relates generally to an indwelling body lumen expander for providing patency to the body lumen. More specifically, the invention relates to one or more indwelling body lumen expanders which may be deployed within a urethral body lumen to provide relief from urinary retention from conditions such as benign prostatic hyperplasia. The expanders and methods described may also be useful in other applications that require expansion of anatomical lumens, e.g., blood vessels, gastrointestinal tract, respiratory tract, reproductive tract, ureters, etc.

### BACKGROUND OF THE INVENTION

Urinary retention occurs when a patient has difficulty or an inability to empty the bladder. There are several causes, but the most common cause in men is benign prostatic hyperplasia (BPH), although urinary retention can also be caused by mechanical blockages from prostate cancer, bladder cancer, urinary tract injury, benign or malignant urethral stricture, etc. Urinary retention is distressing for patients, who may experience urinary hesitancy, straining, weak flow, dripping, incomplete emptying, dysuria, nocturia, frequent urination, and/or incontinence. When a urinary blockage becomes severe, it can cause more serious consequences, including kidney failure or sepsis.

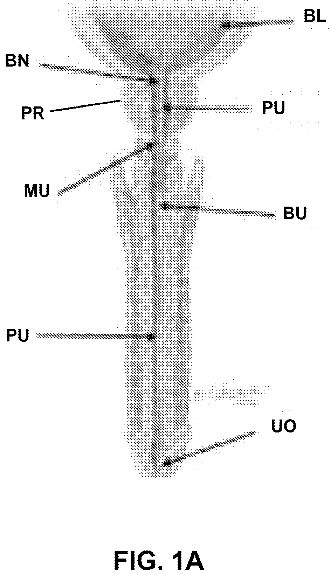

Benign prostatic hyperplasia, also known as prostate enlargement, is the most common cause of obstructive urinary retention. The walnut-sized prostate gland **PR** surrounds the urethra just below the bladder outlet in men, and is known to enlarge with increasing age, as shown in the illustration of Fig. 1A. The bladder outlet is shown at the bladder neck **BN** opening into the prostatic urethra **PU** which extends through the prostrate **PR.** The prostatic urethra extends into the membranous urethra **MU** and further into the bulbar urethra **BU.** The portion of the urethra extending through the penis is shown as the penile urethra **PU** which opens into the urethral opening **UO** at its terminal end.

As the prostate **PR** encroaches on the urethra, the prostate **PR** may begin to obstruct the flow of urine from the bladder **BL** causing discomfort to the patient. BPH typically develops in men older than forty and has increasing prevalence with age, for instance, 50% of men over fifty and 90% of men over eighty have histological evidence of BPH.

The gold standard treatments for drug-refractory moderate-to-severe BPH are surgical in nature and typically involve removing all or part of the prostate gland **PR.** Treatments include open, laparoscopic, and/or robotic prostatic resections including transurethral resection of the prostate (TURP) and laser prostatectomy (LAP). These procedures, performed under general anesthesia, can result in post-operative pain and prolonged catheterization and carry a high risk of sexual side effects. Less invasive energy therapies include transurethral radiofrequency needle ablation (TUNA), transurethral microwave thermotherapy (TUMT).

Less-invasive mechanical methods, such as prostatic stents (e.g., UroLume^{®}, American Medical Systems, Inc., Minnetonka, MN) have been developed as well. Most stent designs involve a tube of metal or plastic that spans the prostatic urethra **PU.** However, these stents often become excessively epithelialized, encrusted, migrated, or calcified when exposed to the urine stream. This in turn leads to discomfort, inflammation, re-obstruction, and infection.

Accordingly, less-invasive methods and devices for treating conditions such as BPH which do not succumb to the deficiencies above are desirable.

US 2004/078088 A1 discloses a stent for treatment of a body lumen through which a flow is effected on either side of a sphincter, said stent comprising one or more windings and having an inner core substantially covered by an outer core and including a first segment, a second segment, and a connecting member disposed between the segments. When the stent is positioned within a patient's urinary system, the first segment and second segments are located on either side of the external sphincter to inhibit migration of the stent while not interfering with the normal functioning of the sphincter.

US 5,540,713 discloses an apparatus for widening a stenosis in a body cavity, such as an artery, in the bile duct, in the ureter, etc., which in view of a problem-free, reliable and permanent widening of a stenosis is characterized by a memory alloy part having a cylindrical jacket-shaped outer contour, said part radially widening at a transition temperature, which is above ambient temperature, but below body temperature, while maintaining a cylindrical outer contour and at a temperature below the transition temperature the diameter is smaller than that of the vessel.

### SUMMARY OF THE INVENTION

According to the present invention there is provided the apparatus claimed in claim 1. Further aspects and preferred embodiments are defined in the dependent claims. Any aspects, embodiments and examples of the present disclosure which do not fall under the scope of the appended claims do not form part of the invention and are merely provided for illustrative purposes.

The deployment of the one or more expandable structures or scaffolds within the prostatic urethra may be used to maintain patency of the prostatic urethra against the enlarged prostate in order to enable a patient to urinate and thereby provide relief from urinary tract retention arising from conditions such as BPH. Such urethral expanding structures may be deployed within the prostatic urethra and left as an indwelling prosthesis. Moreover, one or more of the expanding structures may be deployed adjacent to one another depending upon the degree of collapse of the prostatic urethra as well as the size and length of the prostatic urethra as well.

Generally, such an apparatus for maintaining patency of a body lumen may comprise an expander structure defining an opening and configured for contact against a tissue lining of a body lumen, wherein the expander structure has a diameter which sized to be, e.g., 0% to 60% larger than a diameter of the body lumen, and wherein the expander structure has a height which is configured to be invaginated within the tissue lining of the body lumen while maintaining patency of the body lumen.

In use, one method for maintaining patency of a body lumen may generally comprise advancing the least one expander structure which defines an opening into proximity of a body lumen which is to be maintained in an open configuration, deploying the at least one expander structure against a tissue lining of the body lumen such that the at least one expander structure has a diameter in a deployed configuration which is between, e.g., 0% and 60% larger than the typical diameter of the body lumen. Once expanded, the method may further comprise maintaining a position of the at least one expander structure within the body lumen such that the at least one expander structure is partially or completely invaginated within the tissue lining of the body lumen while maintaining patency of the body lumen.

The various expander structures described are designed to expand the cross-sectional area of the prostatic urethra to allow urine drainage from the bladder. These expanders may be deployed to span the prostatic urethra and may be optionally anchored at their respective locations to prevent migration of the structure after deployment in the patient body. The deployed diameter of the expander structures may be configured to expand to a deployment diameter which is generally larger than that of the urethra.

The one or more expander structures may also optionally incorporate a tissue anchoring mechanism which allows for the securement of the deployed expander directly into the surrounding luminal tissue walls to prevent migration of the structures. Moreover, the expander structures may be designed to have a height (the length of the expander along the luminal tissue wall) and width (wall thickness) which are sufficiently thin enough to allow for the luminal tissue to invaginate the expanders at least partially or completely into the urethral wall. The thinness of the expanders may facilitate the enfolding and/or epithelialization of the urethral tissue wall at least partially or completely around the expanders so that the structures remain out of the urine stream and are thereby protected from calcification or encrustation by the passing urine stream.

Furthermore, the expanders may also optionally incorporate any number of surface textures or features such as openings along the surface of the expanders which may facilitate the invagination of the tissue wall and/or epithelialization. Moreover, the expanders may also optionally incorporate any number of eluting biologic agents, e.g., alpha-blockers, 5-alpha reductase inhibitors, phosphodiesterase-5 inhibitors, etc. which may inhibit further tissue growth. Alternatively, the expanders may also optionally incorporate any number of eluting biologic agents which block cell proliferation, e.g., paclitaxel, sirolimus, zirolimus, etc.

In other variations, the expander may be coated with or integrate a layer of material which is hydrophobic or superhydrophobic to further discourage fluid exposure of the expander structure to the urine.

Additionally, the expander may also optionally incorporate various mechanisms for emitting any number of forms of energy or the expanders may be used along with various forms of energy which may be administered separately from the expander. For instance, an expander may administer thermal, electrical, microwave, mechanical, etc. energy to the tissue prior to, during, or after deployment of the expander. The source of the energy may be controlled or delivered externally from the patient body, e.g., via ultrasound or magnetic induction.

Additionally, the expanders may be comprised of any number of biocompatible materials, e.g., metals such as Nitinol or stainless steel, plastics, polymers such as silicone, PET, PTFE, etc., etc., which may be utilized in any number of combinations either within a single expander or between several expanders utilized together. While the expander structure may remain within the tissue walls, portions of the expander or the entire expander structure may be optionally configured to biodegrade or bioabsorb over a specified period of time.

In the event that the expander is comprised of a shape memory alloy such as Nitinol, the expander may be configured to have a predetermined diameter and shape. Prior to and during deployment of the expander within the body lumen, the expander may have a collapsed low-profile configuration suitable for uninhibited delivery into the body lumen. Once the expander has been desirably positioned, the expander may be allowed to self-expand into its deployment configuration as it heats to body temperature, e.g., above 30° C. Alternatively, the expander may be actuated to expand into its deployed configuration where it may then fully expand into deployment. In yet additional variations, a heating or cooling element (e.g., heated or cooled liquid or gas, Peltier junction, etc.) may be deployed with a deployment instrument or separate from the expander to adjustably heat or cool the expander to allow for its re-configuration into its deployed configuration.

The urethral expanders may be configured in a number of different embodiments. With any of the different configurations, a single expander may be deployed within the lumen or several expanders (e.g., 2 to 20 expanders or more) may be deployed adjacent to one another. In other variations, 2 to 4 expanders may be deployed while in other variations, 3 expanders may be deployed. Alternatively, the expanders may be deployed to overlap with respect to adjacent expanders. These several expanders may be deployed in a sequential or connected manner within the prostatic urethra that facilitates an open passageway from the bladder to the membranous urethra.

One variation may be configured to be adjustably sized either prior to deployment or during deployment via an adjustable locking mechanism which may allow for the sliding release and securement of the free ends of the expander member. The locking mechanism with adjustable ends may allow for the diameter of the expander to be adjusted between a specified minimum and maximum length. The first end may define a receiving channel having a number of projections for receiving the second end which may define one or more complementary projections such that the second end may be adjustably slid into the first end to allow for a ratcheted adjustment and securement between the ends.

Another variation may include an expander which defines an opening therethrough. In this variation, the ends of the expander may be secured to one another via a pin or lock which may extend through openings defined in each of the ends. This variation may also incorporate one or more struts which may be coupled at a hub and extend through the opening to support the expanded configuration of the expander once deployed.

Optionally, the expander structure may also incorporate an anchoring mechanism to facilitate securement of the structure to the surrounding tissue and to prevent its migration when in use. While a single tissue anchor may be used, multiple anchors may also be utilized in a uniform or arbitrary pattern along the outer surface of the expander. Moreover, such anchors may be configured in any number of shapes or patterns or projections which help to prevent expander migration, e.g., hook-shaped, barb-shaped, V-shaped, etc. Additionally, the anchors may not only be configured to extend radially form the expander, they may be configured to extend in alternate directions as well. During delivery of the expander within the body lumen, the anchors may be configured to have a low profile to prevent inadvertent engagement with the tissue wall but when the expander is deployed against the tissue wall, the anchors may be reconfigured, e.g., rotated or repositioned, to face the vessel wall upon deployment. The anchors may also be composed of the same material as the expander or they may be composed of another biocompatible material. While the anchors are illustrated in this particular example, such anchors may be optionally incorporated into any of the other variations of expanders as described herein.

In yet another variation of an expander structure, the expander may be configured into a collapsible structure forming a pattern, e.g., a zig-zag, coiled, sinusoidal, or other pattern, defining an opening which may facilitate the collapse and expansion of the expander along its circumference. In this variation, the expander may be expanded to the desired diameter which may then resist its collapse.

Another variation of the expander structure may be formed by telescoping elements comprised of separate sections which may be assembled into any number of polygonal shapes (e.g., square, rectangular, circular, triangular, elliptical, etc.). The expander assembly may form an octagonal shape (although other shapes are possible) where individual receiving sections may form female receiving channels along one or both ends for translatably receiving sections which may form male ends for insertion into the corresponding female channels of alternating receiving sections. The connections between the male and female ends may be configured to be uni-directional (e.g., ratcheting mechanisms within the receiving sections) so as to provide an expander structure which may be expanded to a deployed shape against the tissue walls which then resists collapse. In another variation, the assembly may form a triangular shape having receiving sections between adjustable male structures. Additionally, the assemblies may include any number of anchoring aspects along the external surfaces of the structures (as described herein) to prevent migration of the structures after deployment.

In yet another variation, the expander may be configured into a helical or spiral configuration which defines a lumen through the structure. This expander may be comprised of a single unitary structure which is maintained in a low-profile configuration for delivery within the urethra. Once deployed, the expander may reconfigure into its helical or spiral configuration against the tissue walls. Materials such as superelastic or shape memory alloys like Nitinol may be suitable for such a structure although other materials (as described herein) may also be utilized.

Other variations may include a single expander structure formed of separate ring-like structures which are aligned with respect to one another to define a lumen and which are coupled to one another via connector elements. The structure may be formed of a wire, ribbon, or other similar structure. However, the ring-like structures may be angled in a non-parallel manner with respect to one another in other variations. The ring-like structures may be formed as individual structures which are then connected to the connector elements although in other variations, the entire structure may be formed of a single element.

Moreover, the structure may include three ring-like structures for positioning along each of the three main lobes of the prostate so that the ring-like structures may become invaginated within the tissues of their respective lobes. Although alternative variations may include a structure having fewer than three or more than three ring-like structures as well as structures which are non-circular in shape but which may include any number of other shapes, as described herein. Other variations may be formed by a single structure which also connects each of the ring-like structures where the ring-like structures are formed by looping the element. Another variation may include connector elements which are parallel to one another but positioned along opposite ends of the ring-like structures. In alternative variations, the connector elements may be positioned at other angles relative to one another instead of being positioned at opposite ends.

Moreover, the relative spacing between the structures may also be varied and the structures themselves may be angled with respect to one another as well. Additionally, any of the structures may optionally incorporate any of the surface modifications such as anchoring mechanisms or various coatings or coverings, as described herein, in any number of various combinations.

In deploying any of the various expander assemblies described herein, various instruments may be employed. One example may include a delivery and deployment instrument having an elongate shaft having a diameter and a length suitable for insertion into the urethra of the patient. The shaft may be formed with a rigid length or partially flexible length so long as the shaft may be advanced, e.g., within the urethral opening and at least partially into the prostatic urethra. The shaft may have an inflatable member such as a balloon positioned near or at the distal end of the shaft which may have one or more expanders positioned upon the balloon in a low profile for intra-luminal delivery. Once the balloon has been suitable positioned within the body lumen, the balloon may be expanded to deploy the one or more expanders into contact against the lumen wall. Once deployed, the balloon may be collapsed for removal from the patient body. Another variation of a shaft may have an umbrella-like expansion mechanism for deploying one or more expanders against the tissue walls. Yet another variation of a deployment instrument may include an inflation reservoir and a tubular expander which may support one or more expanders upon the outer surface of the expander.

The deployment instrument may be advanced and positioned within the body lumen using an intraluminal visualization system, for example, an endoscope or cystoscope which may be integrated with the deployment instrument or separated. The visualization system may be utilized either before or during the deployment procedure. In other variations, an external imaging modality (e.g., ultrasound, computed tomography, magnetic resonance imaging, etc.) may be used in combination with the deployment instrument (and/or intraluminal visualization system). In some variations, the device may be inserted through the working channel of a rigid or flexible cystoscope or cystoscopy sheath, or through channels in other intraluminal imaging tools. In other variations, a balloon or other positioning reference system may be placed, e.g., within the bladder or elsewhere to ensure proper anatomic positioning of the expanders.

The system may also optionally include apparatus and methods for adjustment and/or retrieval of the one or more expanders. One embodiment of this aspect of the system may include a grasper mechanism (e.g., standard grasper tools may be used). Other variations may also include heating or cooling elements to adjust the device to facilitate adjustment and/or removal.

### BRIEF DESCRIPTION OF THE DRAWINGS

Fig. 1A shows an illustration of the urethral anatomy.
Fig. 1B shows an example of one variation of the expanders described herein deployed within the prostatic urethra.
Fig. 1C shows an end view of a single expander having multiple, e.g., three, ring-like structures or coils deployed against the tissue walls of the prostatic urethra.
Fig. 2 shows a top view of one variation of an expander having adjustable ends for sizing the expander to a desired diameter.
Fig. 3 shows a top view of another variation of an expander having a ratcheting mechanism for adjusting the diameter of the expander.
Figs. 4A and 4B show top and perspective views of another variation of an expander having a pin/pinhole mechanism for adjusting the diameter of the expander.
Figs. 5A and 5B show top and perspective views of another variation of an expander having a tissue anchoring mechanism.
Fig. 6 shows a perspective view of another variation of an expander configured to have an adjustably collapsible structure.
Figs. 7A and 7B show top views of expanders having a structure forming a polygonal shape comprised of separate sections which may be adjustably telescoping relative to one another.
Fig. 8 shows a perspective view of an expander formed of a single element having a spiral or helical configuration.
Fig. 9A shows a perspective view of another variation of one or more expanders which are connected by a single spine element.
Fig. 9B shows a perspective view of another variation of one or more expanders formed of a single contiguous element which also forms a single spine.
Fig. 9C shows a perspective view of another variation of one or more expanders connected by spine elements which are aligned in an alternating manner relative to one another.
Fig. 10 shows a perspective view of another variation of one or more expanders which are connected to one another by multiple spine elements aligned in parallel.
Fig. 11 shows a perspective view of another variation of an expander having two ring-like structures or coils connected by a connector element which is longitudinally aligned.
Fig. 12 shows a perspective view of another variation of an expanding having two ring-like structures or coils connected by a curved connector element.
Fig. 13 shows a perspective view of the expander assembly of Fig. 11 deployed adjacent to a second expander assembly within the prostatic urethra.
Fig. 14A shows one variation of a deployment instrument having an inflatable structure such as a balloon for deploying and sizing the expanders.
Fig. 14B shows another variation of a deployment instrument having an umbrella-like expanding mechanism.
Fig. 15 shows another variation of a deployment instrument having an inflation expanding mechanism.
Fig. 16 shows a perspective view of an expander being deployed from a delivery instrument.

### DETAILED DESCRIPTION OF THE INVENTION

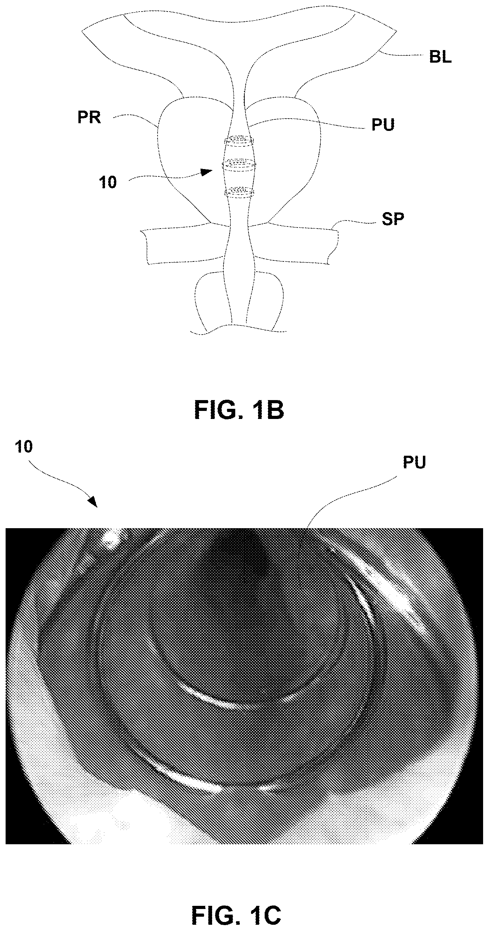

The deployment of one or more expandable structures or scaffolds within the prostatic urethra **PU** may be used to maintain patency of the prostatic urethra **PU** against the enlarged prostate **PR** (shown superior to the sphincter **SP**) in order to enable a patient to urinate and thereby provide relief from urinary tract retention arising from conditions such as BPH. Such urethral expanding structures **10** may be deployed within the prostatic urethra **PU** and left as an indwelling prosthesis, as shown in the side view of Fig. 1B. Moreover, one or more of the expanding structures **10** may be deployed adjacent to one another depending upon the degree of collapse of the prostatic urethra **PU** as well as the size and length of the prostatic urethra **PU** as well, as described herein.

Fig. 1C shows an end view of another example of an expanding structure **10** having three ring-like structures or coils deployed against the interior walls of the prostatic urethra **PU.** Although a single expanding structure **10** is shown, such a structure **10** may have fewer than three or more than three ring-like structures or coils, e.g., two to four, for deployment. Moreover, multiple expanding structures **10** may be deployed within a single urethra, as further described herein.

The description of certain variations of the expanders are not intended to limit the scope of the present invention but other examples, features, aspects, embodiments, and advantages of the invention will become apparent to those skilled in the art from the description herein. Moreover, the expanders and their uses are capable of different and obvious aspects, all without departing from the scope of the invention. Accordingly, the drawings and descriptions should be regarded as illustrative in nature and not restrictive.

The various expander structures described are designed to expand the cross-sectional area of the prostatic urethra **PU** to allow urine drainage from the bladder **BL.** These expanders may be deployed to span the prostatic urethra **PU** and may be optionally anchored at their respective locations to prevent migration of the structure after deployment in the patient body. The deployed diameter of the expander structures may be configured to expand to a deployment diameter which is generally larger than that of the urethra, e.g., the deployed diameter may have a diameter which is, e.g., 0% to 60% or more preferably 10% to 40%, relatively larger than the typical diameter of a prostatic urethra **PU.** While the anatomy of individual patients will vary, the deployment diameter of the expander structure may range anywhere from, e.g., 8 mm to 16 mm. Alternatively, the diameter of a patient's prostatic urethra **PU** may be taken prior to delivery of the expanders and one or more of the expander structures having a diameter which is correspondingly larger in size than the patient's body lumen may be selected for deployment.

The one or more expander structures may also optionally incorporate a tissue anchoring mechanism which allows for the securement of the deployed expander directly into the surrounding luminal tissue walls to prevent migration of the structures. Moreover, the expander structures may be designed to have a height (the length of the expander along the luminal tissue wall) and width (wall thickness) which are sufficiently thin enough to allow for the luminal tissue to invaginate the expanders at least partially or completely into the urethral wall. The thinness of the expanders may facilitate the enfolding of the urethral tissue wall at least partially or completely around the expanders so that the structures remain out of the urine stream and are thereby protected from calcification or encrustation by the passing urine stream. For example, an expander may have a height ranging anywhere from, e.g., 0.2 mm to 1.5 mm, and a width ranging anywhere from, e.g., 0.2 mm to 1.5 mm, so long as the dimensions of the expander facilitate the eventual partial or complete invagination of the expander within the urethral tissue wall. Once one or more expanders have been deployed, partial or complete tissue epithelialization may take anywhere from, e.g., 2 to 8 weeks, given the size of the expanders and the nature of tissue along the urethral wall. The illustration shown in Fig. 1B shows an example of the one or more expanders **10** which have been deployed within the prostatic urethra **PU** of a patient body. The one or more expanders **10** may be seen as having been partially or completely invaginated within the tissue walls of the urethra such that the expanders **10** are no longer in contact with the passing urine but still provide for patency of the prostatic urethra **PU.**

Furthermore, the expanders may also optionally incorporate any number of surface textures or features such as openings along the surface of the expanders which may facilitate the invagination of the tissue wall. For instance, the surface of the expander may be electropolished to decrease friction of the expander during deployment from a delivery instrument. Alternatively, the surface of the expander could also be roughened or otherwise altered to increase friction and anchoring ability in the urethra. In yet another alternative, portions of the expander may be roughened, e.g., the outer surfaces which contact the tissue walls, while the remainder of the surfaces may remain smooth to facilitate delivery and deployment. Roughening the device in this manner could also serve to promote anchoring in the urethra or epithelialization into the urethral wall.

Moreover, the expanders may also optionally incorporate any number of eluting biologic agents, e.g., alpha-blockers, 5-alpha reductase inhibitors, phosphodiesterase-5 inhibitors, etc. which may prohibit additional prostate growth. Alternatively, the expanders may also optionally incorporate any number of eluting biologic agents which block cell proliferation, e.g., paclitaxel, sirolimus, zirolimus, etc.

In other variations, the expander may be coated with or integrate a layer of material which is hydrophobic or superhydrophobic to further discourage fluid exposure of the expander structure to the urine.

Additionally, the expander may also optionally incorporate various mechanisms for emitting any number of forms of energy or the expanders may be used along with various forms of energy which may be administered separately from the expander. For instance, an expander may administer thermal, electrical, microwave, mechanical, etc. energy to the tissue prior to, during, or after deployment of the expander. The source of the energy may be controlled or delivered externally from the patient body, e.g., via ultrasound or magnetic induction.

Additionally, the expanders may be comprised of any number of biocompatible materials, e.g., metals such as Nitinol or stainless steel, plastics, polymers such as silicone, PET, PTFE, etc., etc., which may be utilized in any number of combinations either within a single expander or between several expanders utilized together. While the expander structure may remain within the tissue walls, portions of the expander or the entire expander structure may be optionally configured to biodegrade or bioabsorb over a specified period of time.

In the event that the expander is comprised of a shape memory alloy such as Nitinol, the expander may be configured to have a predetermined diameter and shape. Prior to and during deployment of the expander within the body lumen, the expander may have a collapsed low-profile configuration suitable for uninhibited delivery into the body lumen. Once the expander has been desirably positioned, the expander may be allowed to self-expand into its deployment configuration as it heats to body temperature, e.g., above 30° C. Alternatively, the expander may be actuated to expand into its deployed configuration where it may then fully expand into deployment. In yet additional variations, a heating or cooling element (e.g., heated or cooled liquid or gas, Peltier junction, etc.) may be deployed with a deployment instrument or separate from the expander to adjustably heat or cool the expander to allow for its re-configuration into its deployed configuration.

The urethral expanders may be configured in a number of different embodiments. With any of the different configurations, a single expander (e.g., having one or more ring-like structures or coils) may be deployed within the lumen or several expanders (e.g., 2 to 20 expanders or more) may be deployed adjacent to one another. In other variations, 2 to 4 expanders (e.g., each expander having one or more ring-like structures or coils) may be deployed while in other variations, 3 expanders may be deployed. When deployed, sufficient spacing (e.g., spacing may range anywhere from, e.g., 3 mm to 20 mm) may be provided between adjacent expanders along the prostatic urethra **PU** to allow for the invagination of the expanders by the tissue wall. The spacing between the expanders shall be optimized to permanently relieve the obstruction while minimizing the total amount of material placed in the urethra to avoid tissue irritation or encrustation. Alternatively, the expanders may be deployed to overlap with respect to adjacent expanders. These several expanders may be deployed in a sequential or connected manner within the prostatic urethra **PU** that facilitates an open passageway from the bladder **BL** to the membranous urethra **MU.**

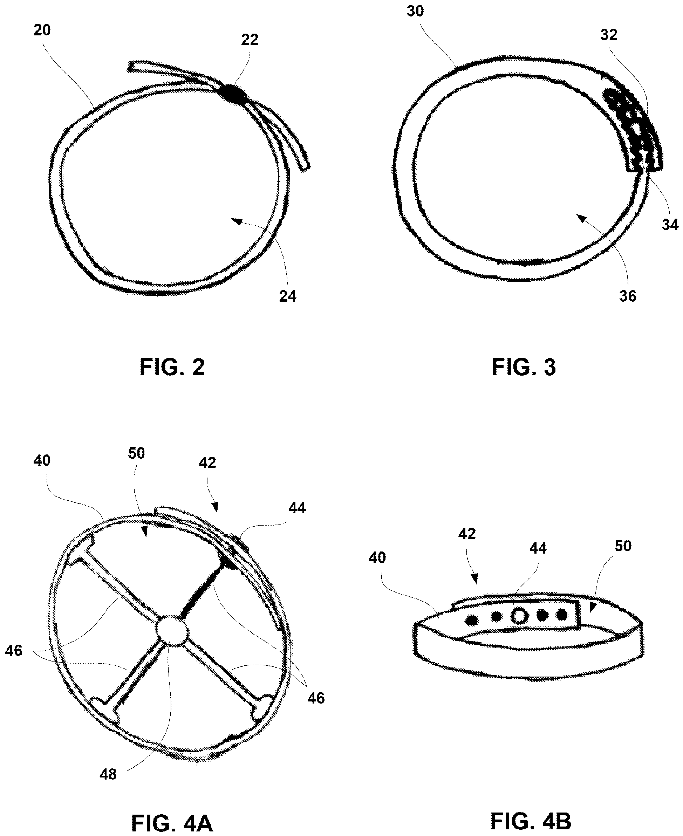

One variation is shown in the top view of Fig. 2 which shows an expander **20** which defines an opening **24** for passage of urine therethrough. The expander **20** may be configured to be adjustably sized either prior to deployment or during deployment via an adjustable locking mechanism **22** which may allow for the sliding release and securement of the free ends of the expander member. The locking mechanism **22** with adjustable ends may allow for the diameter of the expander **20** to be adjusted between a specified minimum and maximum length, as described herein. Fig. 3 shows a top view of another variation of an expander **30** which defines an opening **36** and having a first end **32** which defines an opening for receiving a second end **34**. The first end **32** may define a receiving channel having a number of projections for receiving the second end **34** which may define one or more complementary projections such that the second end **34** may be adjustably slid into the first end **32** to allow for a ratcheted adjustment and securement between the ends.

Another variation is shown in the top and perspective views of Figs. 4A and 4B which illustrate an expander **40** which defines an opening **50** therethrough. In this variation, the ends of the expander **40** may be secured to one another via a pin or lock **44** which may extend through openings **42** defined in each of the ends. This variation may also incorporate one or more struts **46** which may be coupled at a hub **48** and extend through the opening **50** to support the expanded configuration of the expander **40** once deployed.

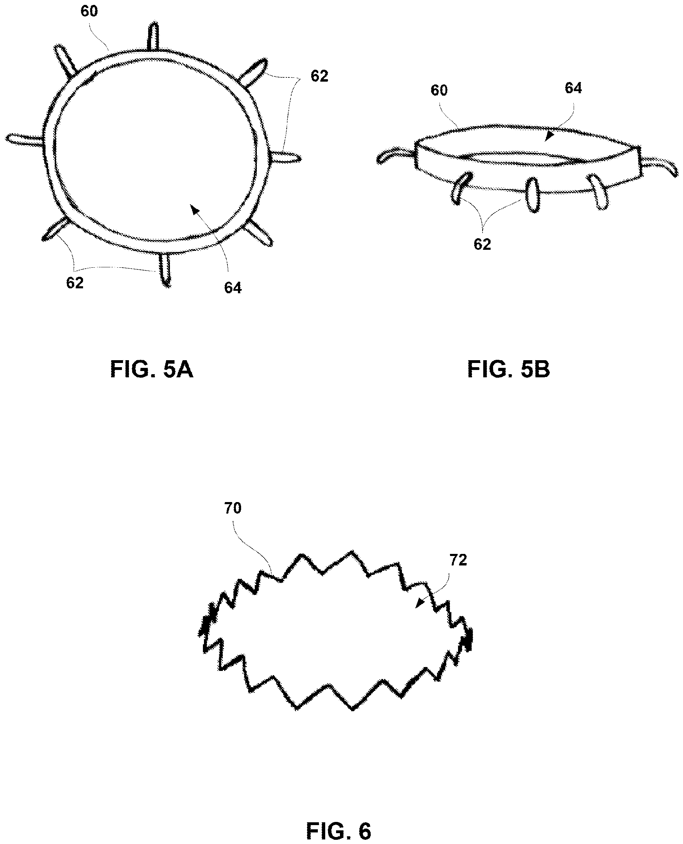

Optionally, the expander structure may also incorporate an anchoring mechanism to facilitate securement of the structure to the surrounding tissue and to prevent its migration when in use. One variation of the anchoring mechanism may be seen in the top and perspective view of Figs. 5A and 5B which show an expander **60** defining an opening **64** and incorporating one or more tissue anchors **62** which extend radially from an outer surface of the expander **60.** While a single tissue anchor **62** may be used, multiple anchors **62** may also be utilized in a uniform or arbitrary pattern along the outer surface of the expander **60.** Moreover, such anchors may be configured in any number of shapes or patterns or projections which help to prevent expander migration, e.g., hook-shaped, barb-shaped, V-shaped, etc. Additionally, the anchors may not only be configured to extend radially form the expander **60,** they may be configured to extend in alternate directions as well. During delivery of the expander **60** within the body lumen, the anchors may be configured to have a low profile to prevent inadvertent engagement with the tissue wall but when the expander **60** is deployed against the tissue wall, the anchors may be reconfigured, e.g., rotated or repositioned, to face the vessel wall upon deployment. The anchors may also be composed of the same material as the expander **60** or they may be composed of another biocompatible material. While the anchors are illustrated in this particular example, such anchors may be optionally incorporated into any of the other variations of expanders as described herein.

In yet another variation of an expander structure, the expander **70** may be configured into a collapsible structure forming a pattern, e.g., a zig-zag, coiled, sinusoidal, or other pattern, defining an opening **72** which may facilitate the collapse and expansion of the expander **70** along its circumference, as shown in the perspective view of Fig. 6. In this variation, the expander **70** may be expanded to the desired diameter which may then resist its collapse.

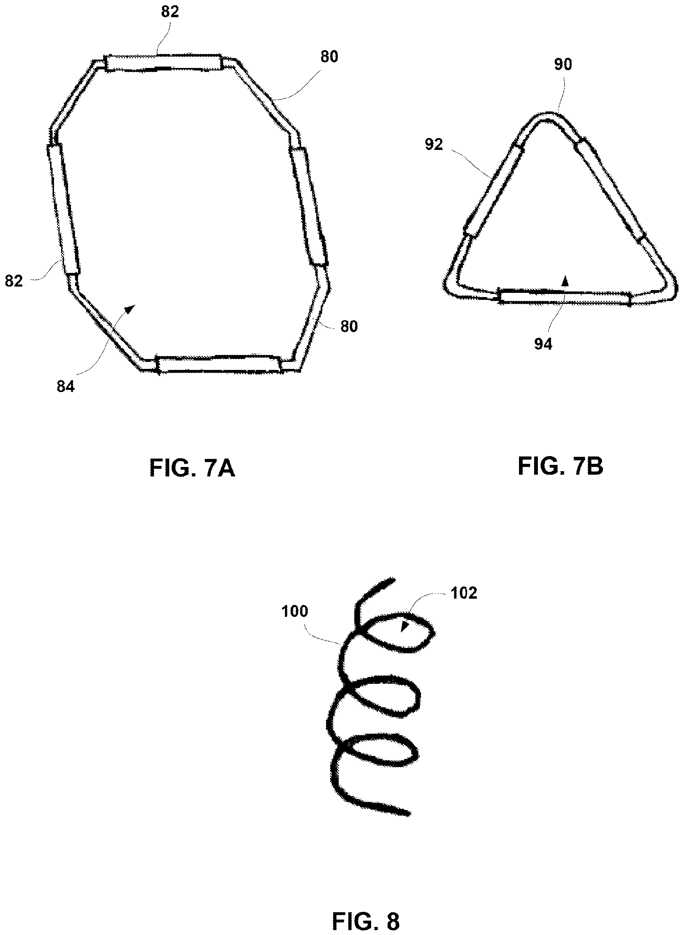

Another variation of the expander structure may be seen in the top views of Figs. 7A and 7B which illustrate structures which are formed by telescoping elements comprised of separate sections which may be assembled into any number of polygonal shapes (e.g., square, rectangular, circular, triangular, elliptical, etc.). Fig. 7A shows one variation of an expander assembly **80** which defines an opening **84.** The expander assembly **80** may form an octagonal shape (although other shapes are possible) where individual receiving sections **82** may form female receiving channels along one or both ends for translatably receiving sections **80** which may form male ends for insertion into the corresponding female channels of alternating receiving sections **82.** The connections between the male and female ends may be configured to be uni-directional (e.g., ratcheting mechanisms within the receiving sections **82**) so as to provide an expander structure which may be expanded to a deployed shape against the tissue walls which then resists collapse. Fig. 7B shows another variation of an expander assembly **90** which defines an opening **94.** In this variation, the assembly **90** may form a triangular shape having receiving sections **92** between adjustable male structures. Additionally, the assemblies may include any number of anchoring aspects along the external surfaces of the structures (as described herein) to prevent migration of the structures after deployment.

In yet another variation, Fig. 8 shows another expander **100** which is configured into a helical or spiral configuration which defines a lumen **102** through the structure. This expander **100** may be comprised of a single unitary structure which is maintained in a low-profile configuration for delivery within the urethra. Once deployed, the expander **100** may reconfigure into its helical or spiral configuration against the tissue walls. Materials such as superelastic or shape memory alloys like Nitinol may be suitable for such a structure although other materials (as described herein) may also be utilized.

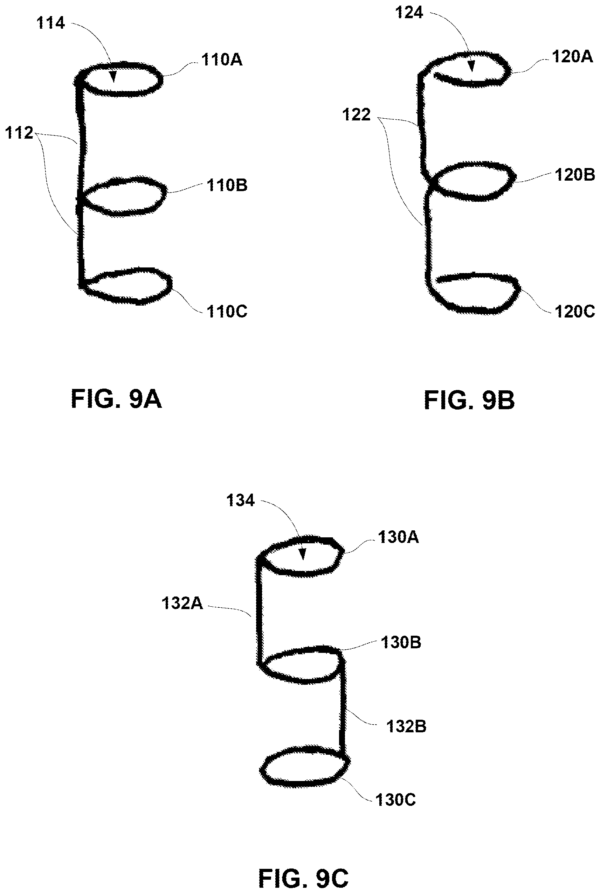

Other variations may be further seen in the perspective views of Figs. 9A to 9C. Fig. 9A shows one variation of a single expander structure assembly formed of separate ring-like structures **110A, 110B, 110C** which are aligned with respect to one another to define a lumen **114** and which are coupled to one another via connector elements **112.** The structure may be formed of a wire, ribbon, or other similar structure having various cross-sectional profiles. For instance, the cross-section of the wire or ribbon may form any number of profile shapes, e.g., circular, elliptical, rectangular, square, trapezoidal, etc. Co-linearly aligned connector elements **112** are shown coupling the ring-like structures **110A, 110B, 110C** along a tangential portion such that the ring-like structures **110A, 110B, 110C** are non-planar and parallel with respect to one another. However, the ring-like structures **110A, 110B, 110C** may be angled in a non-parallel manner with respect to one another in other variations. The ring-like structures **110A, 110B, 110C** may be formed as individual structures which are then connected to the connector elements **112** although in other variations, the entire structure may be formed of a single element.

Furthermore, although these ring-like structures are formed as a single expander assembly, the spacing between the ring-like structures may be similar to the deployed spacing between the individual expanders described herein. The distance between the structures may accordingly range anywhere from, e.g., 3 mm to 20 mm apart from one another to facilitate tissue epithelialization of the structure. Moreover, the distance between adjacent ring-like structures may be applied to any of the various embodiments described herein.

Moreover, the variation shown has three ring-like structures **110A, 110B, 110C** for positioning along each of the three main lobes of the prostate **PR** so that the ring-like structures **110A, 110B, 110C** may become invaginated within the tissues of their respective lobes. Although alternative variations may include a structure having fewer than three or more than three ring-like structures as well as structures which are non-circular in shape but which may include any number of other shapes, as described herein.

Fig. 9B shows another variation where three ring-like structures **120A, 120B, 120C** may define a lumen **124** and which may be formed by a single structure which also connects each of the ring-like structures **120A, 120B, 120C** via connector elements **122.** A single element such as a wire, ribbon, or other similar structure may be used to form a unitary structure where the ring-like structures **120A, 120B, 120C** are formed by looping the element.

Fig. 9C shows a perspective view of yet another variation where the ring-like structures **130A, 130B, 130C** may define a lumen **134** and which are connected by connector elements **132A, 132B.** In this variation, the connector elements **132A, 132B** may be parallel to one another but positioned along opposite ends of the ring-like structures **130A, 130B, 130C.** In alternative variations, the connector elements **132A, 132B** may be positioned at other angles relative to one another instead of being positioned at opposite ends.

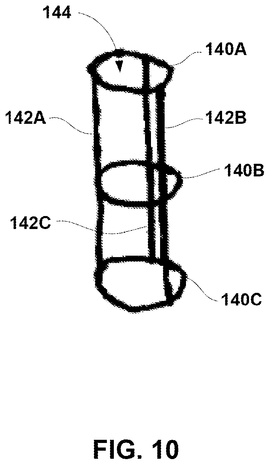

Fig. 10 shows a perspective view of yet another variation where the ring-like structures **140A, 140B, 140C** may define a lumen **144** but in this variation, several connector elements **142A, 142B, 142C** may be arranged in parallel with respect to one another to couple the ring-like structures to one another. Although shown as being uniformly spaced apart from one another in this variation, the connector elements **142A, 142B, 142C** may be spaced at arbitrary positions and may also number two connector elements or more than three connector elements.

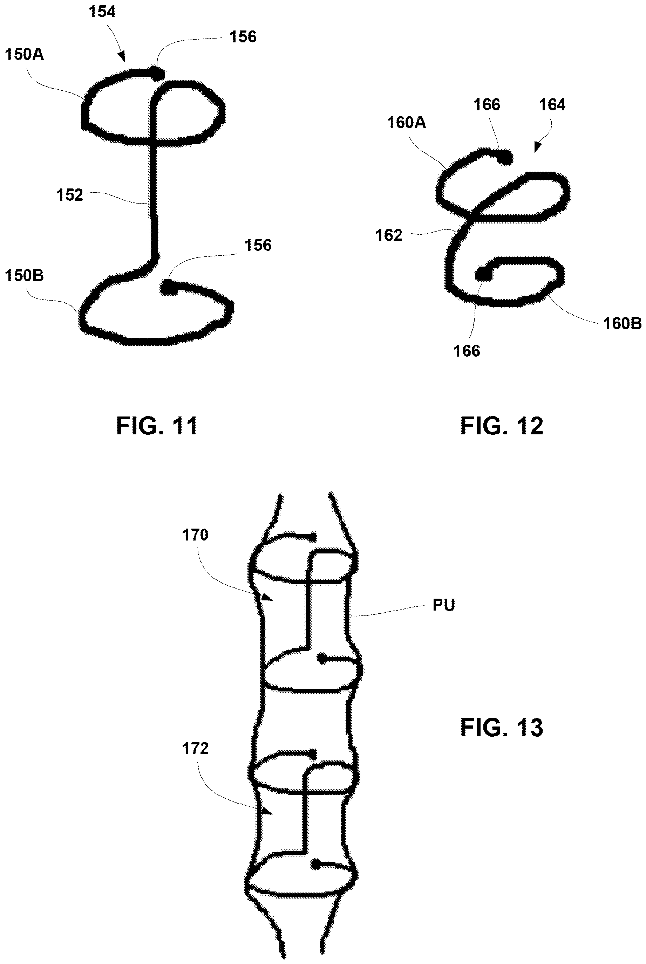

Fig. 11 shows a perspective view of yet another variation of an expander assembly having two ring-like structures **150A, 150B** which define a lumen **154** and are connected by a single connector element **152** which is perpendicularly oriented with respect to the structures **150A, 150B.** When deployed, the ring-like structures **150A, 150B** may be transversely oriented relative to the urethra lumen while the connector element **152** is longitudinally aligned with respect to the urethra lumen. In this variation, the expander assembly may be formed from a single, uniform element which is configured into the expander assembly. Hence, the terminal ends of the ring-like structures **150A, 150B** may be enlarged to present a blunt and atraumatic tip **156** to the surrounding tissue to prevent any perforations.

Fig. 12 shows a perspective view of yet another variation of an expander assembly having two ring-like structures **160A, 160B** which define a lumen **164** and where the structures **160A, 160B** are connected by a single connector element **162.** The element **162** may be curved relative to the structures **160A, 160B** such that the assembly presents a continuously curved or arcuate structure when deployed. The terminal ends of the ring-like structures **160A, 160B** may also be enlarged to present blunt and atraumatic tips **166.**

The blunt and atraumatic tips on the ring-like structure may be optionally incorporated into any of the expander embodiments described herein, as practicable. For instance, the embodiments shown in any of the figures such as Fig. 1C, 8, and 9A to 9C may optionally incorporate such tips.

Fig. 13 shows a perspective view of one example of the expander assembly of Fig. 11 deployed within the prostatic urethra **PU.** In this example, each of the single expanding assemblies **170, 172** may each have two ring-like structures and the expanding assemblies **170, 172** may be deployed adjacent within the urethra to form a continuous structure which may maintain the urethra in an open configuration. As previously described, each of the assemblies **170, 172** may have one or more ring-like structures or coils, e.g., anywhere from two to four, for deployment. Additionally, the spacing between the ring-like structures in expander assembly **170** as well as between the ring-like structure of an adjacent assembly **172** may range anywhere from, e.g., 3 mm to 20 mm apart from one another to facilitate tissue epithelialization of the structure.

Each of the expander configurations shown utilizing three structures (e.g., for positioning against each of the three main lobes of the prostate **PR**) may be altered in other variations to include fewer than three structures or more than three structures. Moreover, the relative spacing between the structures may also be varied and the structures themselves may be angled with respect to one another as well. Additionally, any of the structures may optionally incorporate any of the surface modifications such as anchoring mechanisms or various coatings or coverings, as described herein, in any number of various combinations.

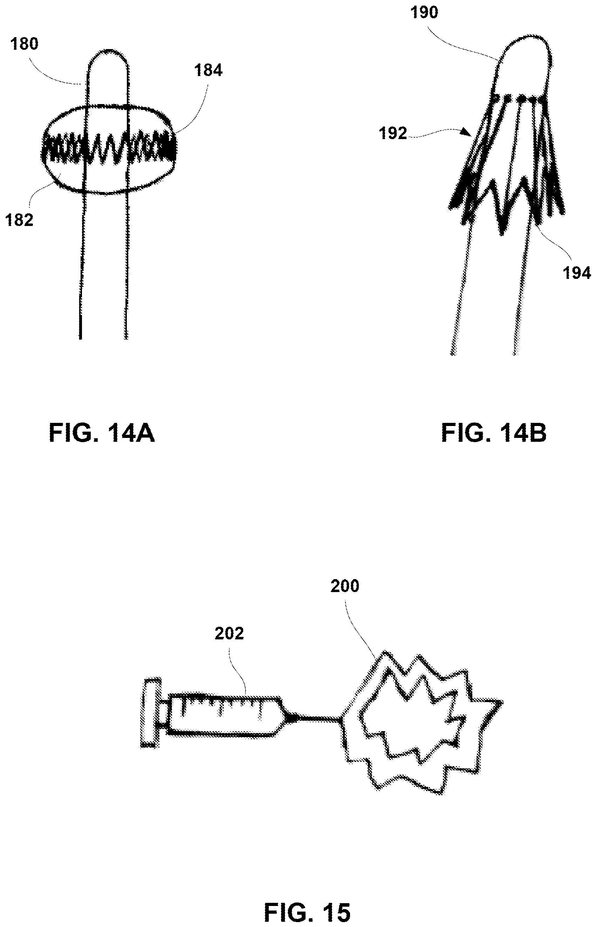

In deploying any of the various expander assemblies described herein, various instruments may be employed. One example is shown in the side view of Fig. 14A which illustrates a delivery and deployment instrument having an elongate shaft **180** having a diameter and a length suitable for insertion into the urethra of the patient. The shaft **180** may be formed with a rigid length or partially flexible length so long as the shaft **180** may be advanced, e.g., within the urethral opening **UO** and at least partially into the prostatic urethra **PU.** The shaft **180** may have an inflatable member such as a balloon **182** positioned near or at the distal end of the shaft **180** which may have one or more expanders **184** positioned upon the balloon **182** in a low profile for intra-luminal delivery. Once the balloon **182** has been suitable positioned within the body lumen, the balloon **182** may be expanded to deploy the one or more expanders **184** into contact against the lumen wall. Once deployed, the balloon **182** may be collapsed for removal from the patient body. Fig. 14B shows a side view of another variation of a shaft **190** having an umbrella-like expansion mechanism **192** for deploying one or more expanders **194** against the tissue walls. Fig. 15 shows a side view of yet another variation of a deployment instrument having an inflation reservoir **202** and a tubular expander **200** which may support one or more expanders upon the outer surface of the expander **200.**

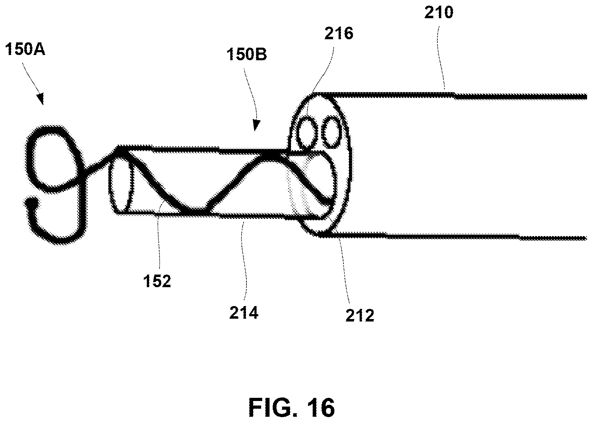

In other variations, as shown in the side view of Fig. 16, the deployment instrument **210** such as an endoscope or cystoscope may define a delivery lumen **212** (e.g., less than 2 mm in diameter) within which the one or more expanders may be positioned while maintained in a low-profile configuration. A pusher or release mechanism may be used to urge the one or more expanders from the delivery lumen **212** in a controlled release once the instrument **210** has been suitably positioned. Moreover, any of the deployment instruments may incorporate any number of steerable components for facilitating advancement of the device within the body lumen. A separate sheath **214** may be advanced or extended through the delivery lumen **212** to help maintain the expander in a low-profile configuration, as shown by the expander assembly and ring-like structures **150A, 150B** as well as connecting element **152** shown in their low-profile delivery configuration. The sheath **214** may be composed of various materials (e.g., PEEK, polyimide, stainless steel, Nitinol, etc.) and may be optimized for strength, maximum inner diameter, and flexibility so as to not inhibit the advancement and/or steering of the instrument **210.** One or several expander assemblies may be positioned within the instrument **210** for sequential deployment such multiple expanders may be deployed in a single procedure.

Once the expander assembly has been delivered from the instrument **210,** the expander may reconfigure itself through its superelastic or shape memory properties into its deployed and expanded configuration into contact against the tissue walls. Alternatively, other actuation mechanisms, as described herein, may be used to reconfigure or facilitate reconfiguration of the expander.

The deployment instrument **210** may be advanced and positioned within the body lumen using an intraluminal visualization system, for example, an endoscope or cystoscope having an imager **216** which may be integrated with the deployment instrument **210** or separated. The visualization system may be utilized either before or during the deployment procedure. In other variations, an external imaging modality (e.g., ultrasound, computed tomography, magnetic resonance imaging, etc.) may be used in combination with the deployment instrument (and/or intraluminal visualization system). In other variations, a balloon or other positioning reference system may be placed, e.g., within the bladder **BL** or elsewhere to ensure proper anatomic positioning of the expanders.

The system may also optionally include apparatus and methods for adjustment and/or retrieval of the one or more expanders. One embodiment of this aspect of the system may include a grasper mechanism (e.g., standard grasper tools may be used). Other variations may also include heating or cooling elements to adjust the device to facilitate adjustment and/or removal.

The applications of the disclosed invention discussed above are not limited to certain treatments or regions of the body, but may include any number of other treatments and areas of the body. Modification of the above-described methods and devices for carrying out the invention, and variations of aspects of the invention that are obvious to those of skill in the arts are intended to be within the scope of this disclosure. Moreover, various combinations of aspects between examples are also contemplated and are considered to be within the scope of this disclosure as well.

## Claims

1. An apparatus (10) for maintaining patency of a prostatic urethra lumen, comprising:
an expander structure (60, 70) defining an opening (64, 72, 114, 124, 134) and configured for contact against a tissue lining of the prostatic urethra lumen,
wherein the expander structure is a unitary structure comprising a plurality of ring-like structures (110A, 110B, 110C, 120A, 120B, 120C, 130A, 130B, 130C) and a plurality of connector elements (112, 122, 132) coupling the ring-like structures along a tangential portion, wherein the plurality of ring-like structures comprises a first, a second, and a third ring-like structure, and wherein only one said connector element (112, 122, 132A) couples the first and second ring-like structures (110A, 110B, 120A, 120B, 130A, 130B) and only one other said connector element (112, 122, 132B) couples the second and third ring-like structures (110B, 110C, 120B, 120C, 130B, 130C);
wherein each of the plurality of ring-like structures is formed by looping the expander structure;
wherein the second ring-like structure transitions to a first one of the connector elements at a first end of the second ring-like structure and transitions to a second one of the connector elements connected at a second end of the second ring-like structure;
wherein the plurality of ring-like structures define a deployment diameter which is sized to be larger than an expanded diameter of the prostatic urethra lumen; and
wherein the expander structure has a height which is configured to be invaginated within the tissue lining of the prostatic urethra lumen while maintaining patency of the prostatic urethra lumen.

2. The apparatus of claim 1, wherein the expander structure comprises an adjustment mechanism for altering a diameter of the structure.

3. The apparatus of claim 1, further comprising one or more anchoring mechanisms (62) defined along the expander structure for facilitating tissue engagement.

4. The apparatus of claim 1, wherein the height of the expander structure is between 0.2 mm to 1.5 mm.

5. The apparatus of claim 1, wherein the expander structure (60, 70) has a width between 0.2 mm to 1.5 mm.

6. The apparatus of claim 1, further comprising one or more additional expander structures, wherein each of the expander structures are spaced 3 mm to 20 mm apart from one another to facilitate tissue invagination.

7. The apparatus of claim 1, wherein the first, second, and third ring- like structures (110A, 110B, 110C, 120A, 120B, 120C, 130A, 130B, 130C) is each a single ring.

8. The apparatus of claim 7, wherein the first, second, and third ring- like structures (110A, 110B, 110C, 120A, 120B, 120C, 130A, 130B, 130C) are parallel to each other.

9. The apparatus of claim 8, wherein the connector elements (112, 122, 132) coupling the first and second ring-like structures (110A, 110B, 120A, 120B, 130A, 130B) and coupling the second and third ring-like structures (110B, 110C, 120B, 120C, 130B, 130C) are perpendicularly oriented with respect to the first, second, and third ring-like structures.

10. The apparatus of claim 1, wherein the plurality of ring-like structures comprises only the first, second, and third ring-like structures (110A, 110B, 110C, 120A, 120B, 120C, 130A, 130B, 130C).

## Patentansprüche

1. Vorrichtung (10) zur Aufrechterhaltung der Durchgängigkeit eines prostatischen Harnröhrenlumens, umfassend:
eine Expanderstruktur (60, 70), die eine Öffnung (64, 72, 114, 124, 134) definiert und für den Kontakt mit einer Gewebeauskleidung des prostatischen Harnröhrenlumens konfiguriert ist,
wobei die Expanderstruktur eine einheitliche Struktur ist, die eine Vielzahl von ringartigen Strukturen (110A, 110B, 110C, 120A, 120B, 120C, 130A, 130B, 130C) und eine Vielzahl von Verbindungselementen (112, 122, 132) umfasst, die die ringartigen Strukturen entlang eines tangentialen Abschnitts verbinden, wobei die Vielzahl der ringartigen Strukturen eine erste, eine zweite und eine dritte ringartige Struktur umfasst, und wobei nur eines der Verbindungselemente (112, 122, 132A) die ersten und zweiten ringartigen Strukturen (110A, 110B, 120A, 120B, 130A, 130B) koppelt und nur ein anderes der Verbindungselemente (112, 122, 132B) die zweiten und dritten ringartigen Strukturen (110B, 110C, 120B, 120C, 130B, 130C) koppelt;
wobei jede der Vielzahl von ringartigen Strukturen durch Schleifenbildung der Expanderstruktur gebildet wird;
wobei die zweite ringartige Struktur an einem ersten Ende der zweiten ringartigen Struktur in ein erstes der Verbindungselemente übergeht und an einem zweiten Ende der zweiten ringartigen Struktur in ein zweites der Verbindungselemente übergeht;
wobei die Vielzahl der ringartigen Strukturen einen Entfaltungsdurchmesser definieren, der größer als ein erweiterter Durchmesser des prostatischen Harnröhrenlumens ist; und
wobei die Expanderstruktur eine Höhe hat, die dafür konfiguriert ist, in die Gewebeauskleidung des prostatischen Harnröhrenlumens eingedrückt zu werden, während die Durchgängigkeit des prostatischen Harnröhrenlumens erhalten bleibt.

2. Vorrichtung nach Anspruch 1, wobei die Expanderstruktur einen Einstellmechanismus zur Veränderung des Durchmessers der Struktur umfasst.

3. Vorrichtung nach Anspruch 1, die ferner einen oder mehrere Verankerungsmechanismen (62) umfasst, die entlang der Expanderstruktur definiert sind, um den Eingriff in das Gewebe zu ermöglichen.

4. Vorrichtung nach Anspruch 1, wobei die Höhe der Expanderstruktur zwischen 0,2 mm und 1,5 mm beträgt.

5. Vorrichtung nach Anspruch 1, wobei die Expanderstruktur (60, 70) eine Breite zwischen 0,2 mm und 1,5 mm aufweist.

6. Vorrichtung nach Anspruch 1, die ferner eine oder mehrere zusätzliche Expanderstrukturen umfasst, wobei jede der Expanderstrukturen 3 mm bis 20 mm voneinander beabstandet ist, um die Gewebeinvagination zu ermöglichen.

7. Vorrichtung nach Anspruch 1, wobei die erste, zweite und dritte ringartige Struktur (110A, 110B, 110C, 120A, 120B, 120C, 130A, 130B, 130C) jeweils ein einzelner Ring ist.

8. Vorrichtung nach Anspruch 7, wobei die erste, zweite und dritte ringartige Struktur (110A, 110B, 110C, 120A, 120B, 120C, 130A, 130B, 130C) parallel zueinander sind.

9. Vorrichtung nach Anspruch 8, wobei die Verbindungselemente (112, 122, 132), die die erste und zweite ringartige Struktur (110A, 110B, 120A, 120B, 130A, 130B) koppeln und die zweite und dritte ringartige Struktur (110B, 110C, 120B, 120C, 130B, 130C) koppeln, senkrecht in Bezug auf die erste, zweite und dritte ringartige Struktur ausgerichtet sind.

10. Vorrichtung nach Anspruch 1, wobei die Vielzahl ringartiger Strukturen nur die ersten, zweiten und dritten ringartigen Strukturen (110A, 110B, 110C, 120A, 120B, 120C, 130A, 130B, 130C) umfasst.

## Revendications

1. Appareil (10) pour maintenir la perméabilité d'une lumière d'urètre prostatique, comprenant :
une structure dilatateur (60, 70) définissant une ouverture (64, 72, 114, 124, 134) et configurée pour entrer en contact contre un revêtement tissulaire de la lumière d'urètre prostatique,
dans lequel la structure dilatateur est une structure unitaire comprenant une pluralité de structures de type anneau (110A, 110B, 110C, 120A, 120B, 120C, 130A, 130B, 130C) et une pluralité d'éléments raccords (112, 122, 132) couplant les structures de type anneau le long d'une partie tangentielle, dans lequel la pluralité de structures de type anneau comprend une première, une deuxième, et une troisième structure de type anneau, et dans lequel seulement un dit élément raccord (112, 122, 132A) couple les première et deuxième structures de type anneau (110A, 110B, 120A, 120B, 130A, 130B) et seulement un autre dit élément raccord (112, 122, 132B) couple les deuxième et troisième structures de type anneau (110B, 110C, 120B, 120C, 130B, 130C) ;
dans lequel chacune de la pluralité de structures de type anneau est formée en bouclant la structure dilatateur ;
dans lequel la deuxième structure de type anneau effectue une transition à un premier des éléments raccords à une première extrémité de la deuxième structure de type anneau et effectue une transition à un second des éléments raccords raccordé à une seconde extrémité de la deuxième structure de type anneau ;
dans lequel la pluralité de structures de type anneau définissent un diamètre de déploiement qui est dimensionné pour être plus grand qu'un diamètre dilaté de la lumière d'urètre prostatique ; et
dans lequel la structure dilatateur a une hauteur qui est configurée pour être invaginée à l'intérieur du revêtement tissulaire de la lumière d'urètre prostatique tout en maintenant la perméabilité de la lumière d'urètre prostatique.

2. Appareil selon la revendication 1, dans lequel la structure dilatateur comprend un mécanisme d'ajustement pour modifier un diamètre de la structure.

3. Appareil selon la revendication 1, comprenant en outre un ou plusieurs mécanismes d'ancrage (62) définis le long de la structure dilatateur pour faciliter l'entrée en prise tissulaire.

4. Appareil selon la revendication 1, dans lequel la hauteur de la structure dilatateur est entre 0,2 mm et 1,5 mm.

5. Appareil selon la revendication 1, dans lequel la structure dilatateur (60, 70) a une largeur entre 0,2 mm et 1,5 mm.

6. Appareil selon la revendication 1, comprenant en outre une ou plusieurs structures dilatateurs supplémentaires, dans lequel chacune des structures dilatateurs est espacée de 3 mm et 20 mm par rapport aux autres pour faciliter l'invagination tissulaire.

7. Appareil selon la revendication 1, dans lequel les première, deuxième, et troisième structures de type anneau (110A, 110B, 110C, 120A, 120B, 120C, 130A, 130B, 130C) sont chacune un seul anneau.

8. Appareil selon la revendication 7, dans lequel les première, deuxième, et troisième structures de type anneau (110A, 11 0B, 110C, 120A, 120B, 120C, 130A, 130B, 130C) sont parallèles les unes aux autres.

9. Appareil selon la revendication 8, dans lequel les éléments raccords (112, 122, 132) couplant les première et deuxième structures de type anneau (110A, 110B, 120A, 120B, 130A, 130B) et couplant les deuxième et troisième structures de type anneau (110B, 110C, 120B, 120C, 130B, 130C) sont orientés perpendiculairement par rapport aux première, deuxième, et troisième structures de type anneau.

10. Appareil selon la revendication 1, dans lequel la pluralité de structures de type anneau comprend seulement les première, deuxième, et troisième structures de type anneau (110A, 110B, 110C, 120A, 120B, 120C, 130A, 130B, 130C).
